# EUROPÄISCHE PATENTSCHRIFT

(11) **EP 1 256 577 B1**
(45) Veröffentlichungstag und Bekanntmachung des Hinweises auf die Patenterteilung: **21.07.2010**
(21) Anmeldenummer: 02009995.8
(22) Anmeldetag: 04.05.2002
(51) Int. Cl.: C07D 251/28

(54) **Verfahren zur Herstellung von Cyanurchlorid**
Process for the preparation of cyanuric chloride
Procédé pour la préparation de chlorure de cyanuryle

(30) Priorität: 11.05.2001 DE 10123072
(43) Veröffentlichungstag der Anmeldung: 13.11.2002
(73) Patentinhaber: Evonik Degussa GmbH, 45128 Essen (DE)
(72) Erfinder: Puschner, Kurt, 63517 Rodenbach (DE); Schauhoff, Stephanie, Dr., 60385 Frankfurt (DE)

(56) Entgegenhaltungen:
- DE-A- 2 843 380
- DE-A- 2 843 381
- DE-A- 2 843 382

## Beschreibung

Die vorliegende Erfindung ist auf ein Verfahren zur Herstellung von Cyanurchlorid gerichtet. Insbesondere betrifft die Erfindung ein Verfahren bei der Cyanurchlorid in einer ersten Einheit synthetisiert, in einer zweiten Einheit gereinigt und in einer dritten Einheit isoliert wird.

Cyanurchlorid ist das Trimerisierungsprodukt von Chlorcyan und ein interessantes Intermediat für die industrielle Weiterverarbeitung zu Farbstoffen, Produkten für die Textilindustrie sowie Pharmazeutika und Pflanzenschutzmitteln.

In den Schriften DE2843381 und DE2843380 wird eine Vorrichtung und ein Verfahren zur Herstellung von Cyanurchlorid offenbart, bei dem nach der Trimerisierung von Chlorcyan das Reaktionsgasgemisch in eine Kolonne ausgestattet mit einem Kondensator am oberen Ende einleitet wird. Die Kondensatortemperatur soll hiernach unterhalb des Siedepunkts des Cyanurchlorids liegen (146-190°C) und das Einleiten des Reaktionsgases erfolgt in der Mitte der Kolonne. Am Boden dieser Kolonne befindet sich eine Heizeinrichtung in der flüssiges Cyanurchlorid kontinuierlich in den Gaszustand überführt wird. Gleichzeitig weist die hier beschriebene Kolonne eine Austragsmöglichkeit an deren unterem Ende auf, durch die flüssiges Material dem System entzogen werden kann. Am oberen Ende der Kolonne ist eine Vorrichtung zum Ableiten gasförmigen Cyanurchlorids mit anschließender Desublimations- und Isoliereinrichtung vorgesehen. Die Lehre dieser Dokumente richtet sich jedoch auf den Aspekt, daß im Verhältnis mehr flüssiges Cyanurchlorid am Boden der Kolonne ausgetragen als gasförmiges über den Kondensator abgeleitet wird. Dies wird unterstrichen durch die vorgeschriebene Kondensatortemperatur von unter 190°C, die eine höhere Abgabe an gasförmigem Cyanurchlorid nicht zuläßt.

Die Güte eines Herstellungsverfahrens für Cyanurchlorid bemißt sich im wesentlichen an der erzeugten Qualität des Herstellungsproduktes und an den gegebenen ökonomischen wie ökologischen Randbedingungen, unter denen das Produkt gewonnen wird.
So bilden die Rieselfähigkeit und Reinheit des gewonnenen Cyanurchlorids - insbesondere für dessen Einsatz zur Herstellung von Pharmazeutika - wichtige Spezifikationskriterien. Weiterhin ist u.a. die Standzeit der eingesetzten Kohle ein entscheidender Parameter für die Berechnung der Kosten des Produkts, bedingt deren Wechsel doch jeweils kostenintensive Stillstandszeiten, in denen kein Produkt in der Anlage hergestellt werden kann (DE19918245).

Aufgabe der vorliegenden Erfindung war deshalb die Angabe eines weiteren Verfahrens, das den eben genannten Punkten in Summe möglichst optimal Rechnung trägt.

Diese und weitere nicht genannte, sich jedoch aus dem Stand der Technik in naheliegender Weise ergebende Aufgaben werden durch ein Verfahren mit den Merkmalen des Anspruchs 1 gelöst. Weiter bevorzugte Merkmale des erfindungsgemäßen Verfahrens werden in den von Anspruch 1 abhängigen Unteransprüchen beschrieben.

Dadurch, daß bei einem Verfahren zur Herstellung von Cyanurchlorid aufweisend eine erste Einheit zur Trimerisierung von Chlorcyan, eine zweite Einheit zur partiellen Kondensation des Cyanurchlorids und eine dritte Einheit zur Desublimation und/oder Kondensation des gasförmigen Cyanurchlorids, ein Reaktionsgasstrom aus der ersten Einheit in den unteren Bereich der zweiten Einheit aufweisend eine Quenchkolonne, welche am oberen Ende einen Kondensator und am unteren Ende eine Austragsmöglichkeit aufweist, geleitet, das den Kondensatorbereich durchströmte gasförmige Cyanurchloridgemisch abgeleitet und anschließend in der dritten Einheit desublimiert und/oder kondensiert wird, wobei man den Kondensator der zweiten Einheit auf eine Temperatur einstellt, bei der mehr Cyanurchlorid gasförmig abgeleitet als kondensiert am Boden der Quenchkolonne ausgetragen wird, gelangt man in überraschend einfacher, dafür aber nicht minder vorteilhafter Art und Weise in hohen Ausbeuten zu einem gleichbleibend qualitativ hochwertigen festen oder wahlweise flüssigen Cyanurchlorid.

Das hier beschriebene Verfahren gestattet es durch Regelung der Kondensatortemperatur das Verhältnis an reinem gasförmigen zu verunreinigtem flüssigen Cyanurchlorid variabel einzustellen. Diese schon aus der DE2843381 und DE2843380 bekannte Tatsache wird jedoch dadurch modifiziert, daß man den Kondensator auf eine Temperatur einstellt, bei der mehr Cyanurchlorid gasförmig abgeleitet als kondensiert am Boden der Quenchkolonne ausgetragen wird, was der Lehre der eben genannten Schriften nicht zu entnehmen ist. Gerade wegen dieser erfindungsmäßen Modifikation erhält man wahlweise ein festes oder flüssiges Produkt, welches zum einen gegenüber dem in DE2843380 und DE2843381 gewonnenen flüssigem Material eine höhere Reinheit aufweist und diese zum anderen durch variable Einstellung der Kondensatortemperatur auch über die Standzeit der Kohle gleichbleibend gut gehalten werden kann. Darüberhinaus toleriert das erfindungsgemäße Verfahren einen mit mehr Verunreinigungen versehenen Reaktionsgasstrom aus der ersten Einheit als im Stand der Technik bei vergleichbarer Qualität möglich, da die Abreicherung der unerwünschten Nebenkomponenten in der zweiten Einheit überraschend effektiv ist. So kann gleichzeitig die Standzeit der Kohle erhöht werden, was wie eingangs erwähnt Vorteile im Hinblick auf die Produktionskosten mit sich bringt.

Wie schon angedeutet kann die Kondensatortemperatur individuell geregelt werden, jedoch muß sie so eingestellt sein, daß mehr Cyanurchlorid gasförmig aus der zweiten Einheit abgeleitet als kondensiert am Boden der Quenchkolonne ausgetragen werden kann. Vorzugsweise sollte die Temperatur jedoch nicht zu hoch gewählt werden, da speziell der Hochsiederanteil bestehend im wesentlichen aus Cyamelurchlorid, Bis-2,6-dichlor-s-triazinyl, tetramerem Chlorcyan, 2,4,5,6 Tetrachlorpyrimidin und 2-Cyanodichlortriazin im abgeleiteten gasförmigen Produkt mit zunehmender Kondensatortemperatur steigt. Im allgemeinen sind Temperaturen des Kondensators auf einen Wert von >der Siedetemperatur des Cyanurchlorids bei Umgebungsdruck einzustellen, bevorzugt >193°C und <200°C. Ganz besonders bevorzugt ist ein Temperaturbereich von >194°C und <198°C einzustellen.

Im Prinzip steht es dem Fachmann offen, wie er die einzelnen Einheiten der Vorrichtung für das erfindungsgemäße Verfahren miteinander verbindet, sofern die Funktionsweise erfindungsgemäß ausgeführt werden kann. So kann es von Vorteil sein, zwischen der ersten Einheit und der zweiten Einheit der Vorrichtung weitere Vorrichtungen zur Reinigung (A-Kohlefilter), Nachreaktion, Temperatureinstellung (Wärmetauscher) etc. vorzusehen, welche helfen, die Produktreinheit und die Ausbeute weiter zu steigern. Bevorzugt ist jedoch die Ausführungsform, bei der der Reaktionsgasstrom direkt aus der ersten Einheit in die zweite Einheit geleitet wird. Dies ist dadurch begründet, daß der besagte Strom beim Verlassen der ersten Einheit derart aufgeheizt ist, daß er beim direkten Einleiten in die zweite Einheit eine Temperatur von bis zu 300°C aufweist. Dadurch wird das Gemisch am Boden der zweiten Einheit (Quenchkolonne) soweit erhitzt, daß das flüssige Cyanurchlorid kontinuierlich in die Gasphase überführt wird. Diese bevorzugte Verfahrensweise erlaubt es also, die zweite Einheit derart auszubilden, daß sie keine zusätzliche Heizquelle aufzuweisen braucht. Der apparative Vorteil dieser Vorrichtung liegt auf der Hand, wird doch durch diese Maßnahme eine zusätzliche Heizquelle am Boden der Quenchkolonne wie in DE2843380 und DE2843381 beschrieben überflüssig.

Wie schon angedeutet soll das über den Kondensator abgeleitete gasförmige Cyanurchlorid die am Boden der Quenchkolonne auszutragende Menge des Produkts übertreffen. Das Verhältnis kann in diesem Bereich vom Fachmann willkürlich mit beschriebenen Maßnahmen eingestellt werden, doch sollte das Mengenverhältnis so eingestellt sein, daß eine erfindungsgemäß gewünschte gleichbleibend gute Reinheit des Cyanurchlorids erhalten wird. Insbesondere sollten das Massenverhältnis von gasförmig abgeleitetem zu am unteren Ende der Quenchkolonne ausgetragenem Cyanurchlorid zwischen 90:10 und 99,9:0,1, vorzugsweise 98:2 und 99:1, betragen.

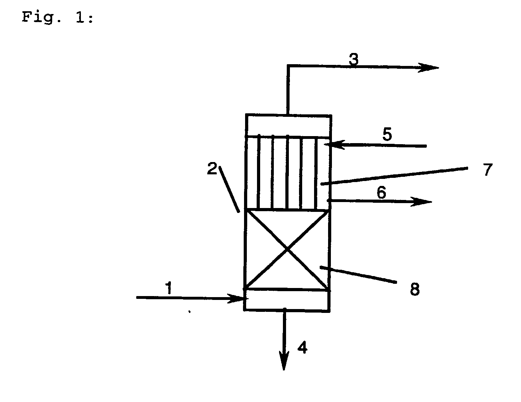

Das erfindungsgemäße Verfahren wird also derart betrieben, daß in einer ersten Einheit die Trimerisierungsreaktion stattfindet. Diese erste Einheit kann nach dem Fachmann geläufigen Merkmalen gestaltet sein, vorzugsweise ist sie identisch mit der in DE 19918245 erwähnten oder dort zitierten Anlage. Der die erste Einheit verlassende Reaktionsgasstrom wird sodann vorzugsweise direkt in den Boden der zweiten Einheit eingeleitet. Eine schematische Darstellung der zweiten Einheit mit den Produktströmen findet sich in der Figur 1. Das heiße Reaktionsgas bringt das am Boden der zweiten Einheit (Quenchkolonne) befindliche Gemisch zum Sieden, woraufhin bevorzugt Cyanurchlorid in die Gasphase übergeht. Das in der Quenchkolonne aufsteigende cyanurchloridreiche, mit Schwersiedern behaftete Gasgemisch kondensiert partiell am auf oberhalb der Siedetemperatur des Cyanurchlorid eingestellten Kondensator am oberen Ende der Quenchkolonne, wobei der Hauptteil sehr reinen Cyanurchlorids gasförmig den Kondensator verläßt und in die dritte Einheit der erfindungsgemäßen Vorrichtung eingeleitet wird und ein teilkondensierter Strom, der nunmehr an Schwersiedern verhältnismäßig höher angereichert ist zum Boden der Quenchkolonne zurückströmt. Dabei trifft er auf von unten kommendes gasförmiges heißes Reaktionsgas, was dazu führt, das aus dem abwärts gerichteten Strom wieder im Verhältnis mehr Cyanurchlorid verdampft und dem Kondensator zuströmt als umgekehrt. Man erreicht so bei minimalem Ausbeuteverlust an Cyanurchlorid und minimalem apparativen Aufwand ein Maximum an Produktreinheit.

Die zweite Einheit kann dabei nach dem Fachmann bekannten Gesichtspunkten gestaltet sein. Vorzugsweise ist die Quenchkolonne mit Füllkörpern beschickt, was zu einer Erhöhung der Bodenzahl bei der partiellen Kondensation und damit zu einer verbesserten Reinigung des Produktstromes führt. Weitere Ausgestaltungen der zweiten Einheit wie Dimensionierung sowie insbesondere die Art, Anbringung und Auslegung des Kondensatorteils sind dem Fachmann überlassen. Derartige Dinge können darüberhinaus der Literatur entnommen werden (Grundoperationen chemischer Verfahrenstechnik, Vauck/Müller 8. Auflage 1988, S. 490-493; Wärmeatlas, 5. Auflage, 1988, S. Cb8-8 bzw. Ja1-Je20).

Die dritte Einheit, in die der Cyanurchloridstrom vorzugsweise gasförmig zur Desublimation und/oder Kondensation eingeleitet wird, kann ebenfalls vom Fachmann beliebig gestaltet werden. Anregungen zur Ausgestaltung können beispielsweise der DE2843380 und DE2843381 entnommen werden. Auch der Ullmann, 1996, Band 3, 2-18, 2-88, 3-29, 2-85, 5-5, Ullmann Band 2 ,Seite 664-671, Ausgabe 1972 oder Perry Chemical Engineers Handbook, 4. Auflage, Ausgabe 1964, Kapitel 17-23 bis 17-26 sowie die EP137505 beschreiben hier erfolgreich einzusetzende Desublimations- und Kondensationseinrichtungen.

Insgesamt hilft das Verfahren in vielen Belangen eine effizientere und damit kostengünstigere Herstellung des Cyanurchlorids bei verbesserter Reinheit zu gewährleisten. Insbesondere die Tatsache, daß trotz der Erhöhung der Kondensatortemperatur auf oberhalb der in DE2843381 und DE2843380 genannten keine Verschlechterung der Produktreinheit unter die Spezifikationsgrenzen eintritt, bedingt einerseits eine kontinuierlich hohe Ausbeute bei gleichbleibend guter Reinheit des Produktes und führt andererseits zu einer Erhöhung der Standzeit des Katalysators in der Trimerisierungsreaktion. All dies lag zum Zeitpunkt der Erfindung mitnichten nahe.

Neben den im Reaktionsgasstrom vorhandenen und beschriebenen Schwersiedern befinden sich hierin auch Verbindungen, die niedriger sieden als Cyanurchlorid. Es versteht sich von selbst, daß diese mit dem gasförmigen Cyanurchlorid den Kondensator der zweiten Einheit verlassen. Eine Abtrennung dieser Komponenten erfolgt dann in der Desublimations- bzw. Kondensationseinheit nach dem Fachmann geläufiger Manier (DE2843381 und DE2843380, EP137505).

### Beschreibung der Figur 1:

Durch die Zuleitung (1) gelangt der heiße Reaktionsgasstrom aus der ersten Einheit in die zweite Einheit (Quenchkolonne 2). Das Cyanurchlorid durchströmt die Quenchkolonne durch den Kolonnenteil (8), bis es auf den Kondensator (7) stößt. Dieser wird über die Leitung (5) mit entsprechend temperierter Kühlflüssigkeit versorgt, die durch Leitung (6) wieder abfließen kann. Der kondensierte Teil des Gasstromes fließt herab zum Boden von (2) und wird der Kolonne (2) durch die Austragsmöglichkeit (4) entnommen. Am Kopf von (2) wird das Cyanurchlorid über die Leitung (3) in die dritte Einheit weitergeleitet.

### Beispiele:

1) 4,4 kg/h Cyanurchloriddampf mit einer Temperatur von 280°C werden in die Quenchkolonne eingeleitet. Die Konzentration an hochsiedenden Nebenprodukten beträgt 0,41 Gew.-%, dies entspricht einem Strom von ca. 0,018 kg/h. Bei einer eingestellten Temperatur von 194°C für die Partialkondensation ergibt sich eine flüssige Ablaufmenge von 1,42 kg/h mit einem Nebenproduktgehalt von 1,15 Gew.-%, dies entspricht einem Nebenproduktstrom von ca. 0,016 kg/h (90%).
2) Gemäß Beispiel 1 wird die Partialkondensation bei 197°C durchgeführt. Dabei wird eine flüssige Ablaufmenge von 0,66 kg/h mit einem Nebenproduktanteil von 2,41 Gew.-% erhalten. Dies entspricht ebenfalls einem Nebenproduktstrom von 0,016 kg/h (90%).

## Patentansprüche

1. Verfahren zur Herstellung von Cyanurchlorid aufweisend eine erste Einheit zur Trimerisierung von Chlorcyan, eine zweite Einheit zur partiellen Kondensation des Cyanurchlorids und eine dritte Einheit zur Desublimation und/oder Kondensation des gasförmigen Cyanurchlorids, wobei ein Reaktionsgasstrom aus der ersten Einheit in den unteren Bereich der zweiten Einheit aufweisend eine Quenchkolonne, welche am oberen Ende einen Kondensator und am unteren Ende eine Austragsmöglichkeit aufweist, geleitet wird und Ableiten des den Kondensatorbereich durchströmten gasförmigen Cyanurchloridgemisches mit anschließender Desublimation und/oder Kondensation in der dritten Einheit,
**dadurch gekennzeichnet, daß**
man den Kondensator der zweiten Einheit auf eine Temperatur einstellt, bei der mehr Cyanurchlorid gasförmig abgeleitet als kondensiert am Boden der Quenchkolonne ausgetragen wird, wobei man die Temperatur des Kondensators auf einen Wert von >Siedepunkt des Cyanurchlorids bei Umgebungsdruck einstellt.

2. Verfahren nach Anspruch 1,
**dadurch gekennzeichnet, daß** man die Temperatur auf >193°C und <200°C einstellt.

3. Verfahren nach einem oder mehreren der vorhergehenden Ansprüche,
**dadurch gekennzeichnet, daß**
der Reaktionsgasstrom direkt aus der ersten Einheit in die zweite Einheit geleitet wird.

4. Verfahren nach einem oder mehreren der vorhergehenden Ansprüche,
**dadurch gekennzeichnet, daß**
die zweite Einheit keine zusätzliche Heizquelle aufweist.

5. Verfahren nach einem oder mehreren der vorhergehenden Ansprüche,
**dadurch gekennzeichnet, daß**
das Massenverhältnis von gasförmig abgeleitetem zu am unteren Ende der Quenchkolonne ausgetragenem Cyanurchlorid zwischen 90:10 und 99,9:0,1, vorzugsweise 98:2 und 99:1, beträgt.

## Claims

1. Process for producing cyanuric chloride, comprising a first unit for trimerizing cyanogen chloride, a second unit for partially condensing cyanuric chloride and a third unit for desubliming and/or condensing gaseous cyanuric chloride, wherein a reaction gas stream is routed from the first unit into the lower region of the second unit comprising a quenching column which includes a condenser at the upper end and discharge means at the lower end, and removing the gaseous cyanuric chloride mixture which has passed through the condenser region, with subsequent desublimation and/or condensation in the third unit,
**characterized in that** the condenser of the second unit is set to a temperature at which more cyanuric chloride is removed in gaseous form than is discharged in condensed form at the base of the quenching column, and the temperature of the condenser is set to a value of >boiling point of the cyanuric chloride at ambient pressure.

2. Process according to Claim 1, **characterized in that** the temperature is set to >193°C and <200°C.

3. Process according to either or both of the preceding claims, **characterized in that** the reaction gas stream is routed directly from the first unit into the second unit.

4. Process according to one or more of the preceding claims, **characterized in that** the second unit does not include any additional source of heat.

5. Process according to one or more of the preceding claims, **characterized in that** the mass ratio of cyanuric chloride removed in gaseous form to cyanuric chloride discharged at the lower end of the quenching column is between 90:10 and 99.9:0.1, preferably 98:2 and 99:1.

## Revendications

1. Procédé pour la préparation de chlorure de cyanuryle, comprenant une première unité pour la trimérisation de chlorure de cyanogène, une deuxième unité pour la condensation partielle du chlorure de cyanuryle et une troisième unité pour la désublimation et/ou la condensation du chlorure de cyanuryle gazeux, un flux de gaz de réaction sortant de la première unité étant envoyé dans la zone inférieure de la deuxième unité comprenant une colonne de refroidissement qui comporte à l'extrémité supérieure un condenseur et à l'extrémité inférieure une possibilité de décharge, et évacuation du mélange gazeux contenant du chlorure de cyanuryle ayant traversé la zone du condenseur, avec désublimation et/ou condensation subséquente(s) dans la troisième unité,
**caractérisé en ce qu'**on règle le condenseur de la deuxième unité à une température à laquelle est déchargé plus de chlorure de cyanuryle évacué à l'état gazeux que condensé au fond de la colonne de refroidissement, la température du condenseur étant ajustée à une valeur supérieure au point d'ébullition du chlorure de cyanuryle sous la pression de l'environnement.

2. Procédé selon la revendication 1, **caractérisé en ce que** la température est ajustée à >193 °C et <200 °C.

3. Procédé selon une ou plusieurs des revendications précédentes, **caractérisé en ce que** le flux de gaz de réaction sortant de la première unité est envoyé directement dans la deuxième unité.

4. Procédé selon une ou plusieurs des revendications précédentes, **caractérisé en ce que** la deuxième unité ne comporte pas de source de chaleur supplémentaire.

5. Procédé selon une ou plusieurs des revendications précédentes, **caractérisé en ce que** le rapport massique du chlorure de cyanuryle évacué à l'état gazeux au chlorure de cyanuryle déchargé à l'extrémité inférieure de la colonne de refroidissement est compris entre 90:10 et 99,9:0,1, de préférence entre 98:2 et 99:1.
